# EUROPEAN PATENT APPLICATION

(11) **EP 4 640 145 A1**
(43) Date of publication of application: **29.10.2025**
(21) Application number: 25171651.0
(22) Date of filing: 22.04.2025
(51) Int. Cl.: A61B 5/021, A61B 5/022, A61B 5/024, A61B 5/00

(54) **PRESSURE-SENSITIVE SMART ELECTRONIC BRACELET AND APPLICATION METHOD THEREOF**

(30) Priority: 26.04.2024 TW 113115617
(71) Applicant: iRobot Medicine Technology CO., LTD., Taipei City 114 (TW)
(72) Inventor: MI, Hsin-Wu, Taipei City (TW); LIAO, En-Chih, Taipei City (TW); LIN, Tsung-Han, Taipei City (TW); TU, Chang-Hung, Taipei City (TW)
(74) Representative: Becker, Eberhard

(57) **Abstract**

The present invention is a pressure-sensitive smart electronic bracelet (1) and its application method. The smart electronic bracelet comprises a first body (10), a second body (20), and a bracelet band (30). Two opposite sides of the first body (10) are respectively connected to the second body (20) and the bracelet band (30). The first body (10) is equipped with an air pump (12), and the second body (20) is equipped with multiple pressure sensors (24) and an airbag (23), with each pressure sensor (24) having a contact portion (240) and the airbag (23) connected to the air pump (12). When a user wears the bracelet and the airbag (23) is inflated, each pressure sensor (24) receives the user's pulse beats information through the contact portion (240) to accordingly generate a pressure sensing signal (S1).

## Description

### 1. Field of the Invention

The present invention relates to a smart electronic bracelet, especially a smart electronic bracelet and an application method thereof that can detect vital signs, such as blood pressure, pulse, and other physiological indicators, of a user through pressure sensing.

### 2. Description of the Related Art

With developments of science and technology, smartphones are widely used, improving conveniences in daily activities through various functions such as mobile payment, daily vital signs recording, etc. Yet some daily occasions are not conducive for the users to carry smartphones to perform the functions abovementioned. For example, the smartphone is not conducive for the users to carry it around during exercise due to its size and weight, hindering users from recording their vital signs during exercise.

Therefore, wearable smart devices have been invented to facilitate users to perform some smartphone functions through wearable smart devices in specific situations. For example, since an electronic bracelet (the wearable smart device) is more lightweight and compact-sized than a smartphone, the user can wear it for a long time compared with smartphones, allowing the electronic bracelet to record daily physiological data more comprehensively.

For compactness, space for accommodating physiological data sensors in the electronic bracelet is reduced, therefore limiting types of the physiological data sensors. For example, most conventional electronic bracelets mainly rely on optical principles for sensing physiological data. An inner surface of a conventional electronic bracelet is equipped with an optical sensor. When the user wears the said electronic bracelet, the optical sensor will be attached to the user's skin to sense a light intensity signal reflected by user's blood through optical principles. A processing device in the electronic bracelet will compute multiple physiological data, such as pulse condition, blood pressure, body temperature data, etc., according to the light intensity signal. However, the electronic bracelet only computes the multiple physiological data based on the light intensity signal, and measuring accuracy of data such as the pulse condition and the blood pressure will be questionable. Accordingly, traditional electronic bracelet must be improved in terms of the accuracy of physiological data acquired via physical methods, such as pressure-based sensing.

Conventional electronic bracelets mainly rely on optical sensing principles to obtain users' physiological data, such that measuring accuracies of some physiological data are questionable. To overcome the aforementioned issue, the present invention provides a pressure-sensitive smart electronic bracelet and application methods thereof, wherein the smart electronic bracelet detects a user's physiological data through physical sensing technology (pressure sensing) to improve the measuring accuracy of physiological data such as blood pressure and pulse condition.

The pressure-sensitive smart electronic bracelet of the present invention comprises:
a first body, comprising:
   a first shell with a first side and a second side opposite to the first side, the first side connected with a bracelet band and the first shell containing a first accommodating space inside; and
   an air pump positioned in the first accommodating space; and
a second body, comprising:
   a second shell connected with the second side of the first shell with a second accommodating space inside the second shell;
   a control circuit board positioned in the second accommodating space and comprising a first surface and a second surface opposite to the first surface;
   multiple pressure sensing groups, each pressure sensing group comprising multiple pressure sensors positioned on the first surface of the control circuit board, each pressure sensor electrically connected to the control circuit board with a contact portion thereof exposed from an outer surface of the second shell, and each pressure sensor detecting a user's pulse beats information to accordingly generate a pressure sensing signal; and
   an airbag positioned in the second accommodating space, located between the second surface of the control circuit board and the second shell, and connected to the air pump.

One application method of the aforementioned pressure-sensitive smart electronic bracelet is provided, wherein,
when the bracelet is tied to a user's wrist, the air pump of the bracelet inflates the airbag according to the user's operation;
when the air pump stops operating and the airbag is deflating, the control circuit board receives the pressure sensing signal transmitted by each pressure sensor and performs multiple signal processing processes on each pressure sensing signal to obtain a pulse amplitude signal, and the control circuit board transmits the pulse amplitude signal to an AI (Artificial Intelligence) server, which extracts multiple feature point information through deep analysis and computation;
the multiple feature point information includes a systolic blood pressure information and a diastolic blood pressure information.

Another application method of the aforementioned pressure-sensitive smart electronic bracelet is provided, wherein,
when the bracelet is tied to a user's wrist, the air pump of the bracelet inflates the airbag according to the user's operation;
when the air pump stops operating and the airbag is deflating, the control circuit board receives the pressure sensing signal transmitted by each pressure sensor, performs multiple signal processing processes on each pressure sensing signal to obtain a pulse wave signal, performs Fourier transform on the pulse wave signal to obtain a high-frequency signal and a low-frequency signal, and transmits the pulse wave signal, the high-frequency signal, and the lower-frequency signal to an AI server, which extracts multiple feature wave information through deep analysis and computation.

The pressure-sensitive smart electronic bracelet of the present invention comprises a first body and a second body. The present invention, in contrast to prior art, provides increased space for accommodating physiological data sensors while maintaining compatibility with various sensor types. The second body is equipped with multiple pressure sensors each with a contact portion. When the user wears the pressure-sensitive smart electronic bracelet, each pressure sensor receives the user's pulse beats information through the contact portion to accordingly generate a pressure sensing signal. In addition, an airbag is positioned in the second body. When the airbag is inflated, each contact portion can further protrude from the outer surface of the second body to increase the contact area with the user. Each contact portion is pushed by the airbag and more closely attached to the user. The conventional electronic bracelet only senses pulse data through the optical sensor with its transmitting terminal (or a receiving terminal) prone to blockage by objects, such as dust, moisture, etc., resulting in inaccuracy of pulse data sensed by the optical sensor. The pressure-sensitive smart electronic bracelet of the present invention measures the user's pulse data through physical contacting, effectively improving the accuracy of measuring physiological data such as blood pressure and pulse condition.

The pressure-sensitive smart electronic bracelet of the present invention features unique physical sensing technology and can instantly detect the user's vital signs, including blood pressure, pulse condition and other physiological indicators. The pressure-sensitive smart electronic bracelet transmits the obtained data to the AI server, which performs deep analysis and computation to obtain multiple analysis results. The multiple analysis results can be fed back to the user and also simultaneously provided to relevant medical institutions. Consequently, healthcare providers can swiftly gain insight into the user's physiological state, facilitating appropriate medical interventions and advice.

### In the drawings

Fig. 1 illustrates a schematic diagram of an appearance of a pressure-sensitive smart electronic bracelet of the present invention;
Fig. 2 illustrates another schematic diagram of an appearance of the pressure-sensitive smart electronic bracelet of the present invention;
Fig. 3 shows a schematic diagram of wearing the pressure-sensitive smart electronic bracelet of the present invention;
Fig. 4 depicts a cross-sectional side view of a second shell of the pressure-sensitive smart electronic bracelet of the present invention, with an airbag uninflated;
Fig. 5 depicts a cross-sectional side view of the second shell of the pressure-sensitive smart electronic bracelet of the present invention, with the airbag inflated;
Fig. 6 presents a circuit block diagram of the pressure-sensitive smart electronic bracelet of the present invention;
Fig. 7 depicts a cross-sectional front view of the pressure-sensitive smart electronic bracelet of the present invention situated in an electronic bracelet charging cabinet;
Fig. 8 depicts another cross-sectional side view of the pressure-sensitive smart electronic bracelet of the present invention situated in the electronic bracelet charging cabinet;
Fig. 9 shows a schematic diagram of the appearance of an identity pairing device utilized in conjunction with the pressure-sensitive smart electronic bracelet of the present invention;
Fig. 10 shows a schematic diagram of the appearance of an auxiliary wearing frame utilized in conjunction with the pressure-sensitive smart electronic bracelet of the present invention;
Fig. 11 shows a curve schematic diagram of a relationship between pressure and time when the airbag of the pressure-sensitive smart electronic bracelet of the present invention stops inflating;
Fig. 12 shows a curve schematic diagram of a relationship between pressure and time of a pulse amplitude signal computed by the pressure-sensitive smart electronic bracelet of the present invention;
Fig. 13 presents a circuit block diagram of the pressure-sensitive smart electronic bracelet of the present invention and an AI server; and
Fig. 14 shows a schematic diagram of superimposition of an air pressure change curve and the pulse amplitude signal.

In order to understand the technical characteristics and practical effects of the prevent invention in detail, and accomplish them according to the content of the present invention, the detailed description is as follows with the embodiments shown in the figures.

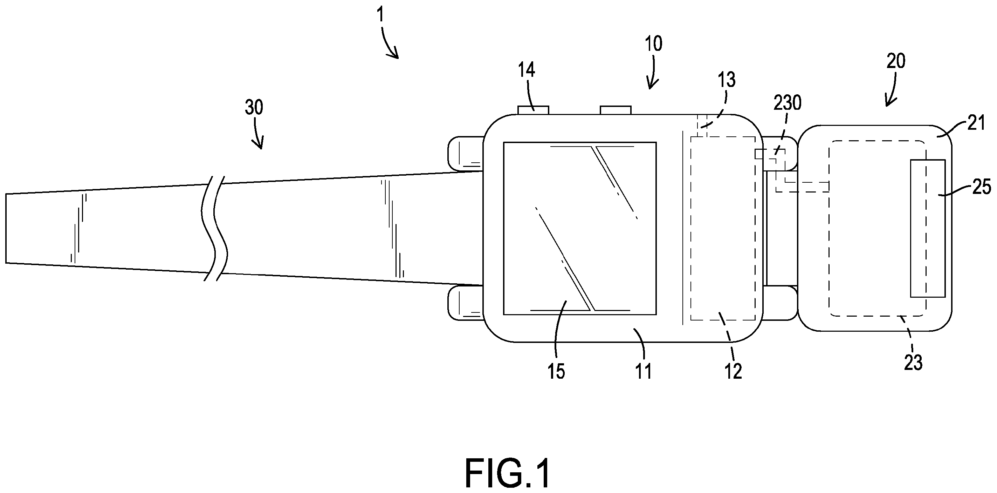

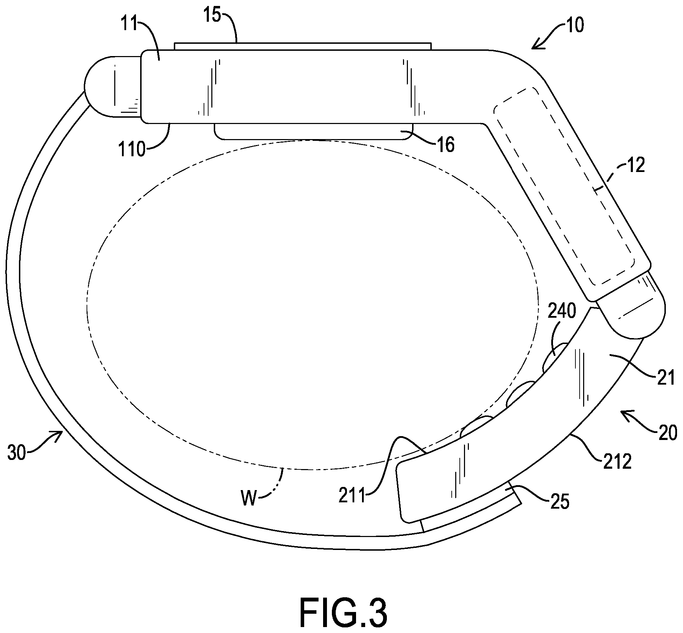

Referring to Figs. 1 to 3, a pressure-sensitive smart electronic bracelet 1 of the present invention comprises a first body 10, a second body 20, and a bracelet band 30. The first body 10 is connected to the second body 20 and the bracelet band 30 respectively. In particular, the second body 20 and the bracelet band 30 are respectively connected to two opposite sides of the first body 10. The bracelet band 30 is detachably connected to the second body 20. When the bracelet band 30 is connected to the second body 20, the pressure sensitive smart electronic bracelet 1 is annular in shape as shown in Fig. 3 for the user to wear.

The first body 10 comprises a first shell 11 and an air pump 12. The first shell 11 has a first side and a second side opposite to the first side. The first side is connected to the bracelet band 30, and the second side is connected to the second body 20. The first shell 11 contains a first accommodating space (not shown in Figs) inside. The air pump 12 is positioned in the first accommodating space. In particular, the first shell 11 is formed with a channel 13 that connects the first accommodating space with the outside of the first shell 11. When the air pump 12 is operated, air outside the first shell 11 can be extracted through the channel 13 for inflation.

In an embodiment of the present invention, the first body 10 also comprises at least one button 14, a screen 15, an optical sensor 16, and a first controller. The first controller is electrically connected with the at least one button 14, the screen 15, the optical sensor 16, and the air pump 12.

The at least one button 14 is positioned on at least one outer surface of the first shell 11 and is operable by the user to perform functions of the pressure-sensitive smart electronic bracelet 1, such as turning on the air pump 12 by pressing the at least one button 14. The screen 15 is positioned in the first accommodating space with a display surface of the screen 15 exposed from the outer surface of the first shell 11; preferably, the screen 15 is a touchscreen. Referring to Fig. 3, when the pressure sensitive smart electronic bracelet 1 is worn on a wrist W of the user, the surface of the first shell 11 that contacts the user is defined as a user contact surface 110. The screen 15 is exposed from the outer surface opposite to the user contact surface 110 and therefore can be operated by the user to perform the functions of the pressure-sensitive smart electronic bracelet 1. For example, in addition to turning on the air pump 12 through the at least one button 14 as aforementioned, the user can also turn on the air pump 12 by touching the screen 15.

The optical sensor 16 is positioned in the first accommodating space with a detecting surface of the optical sensor 16 exposed from the outer surface of the first shell 11. In particular, the detecting surface of the optical sensor 16 is exposed from the user contact surface 110, the optical sensor 16 emitting light and receiving reflected light by the detecting surface to detect the user's physiological data. The first controller is positioned in the first accommodating space and is, for example, a microcontroller (MCU).

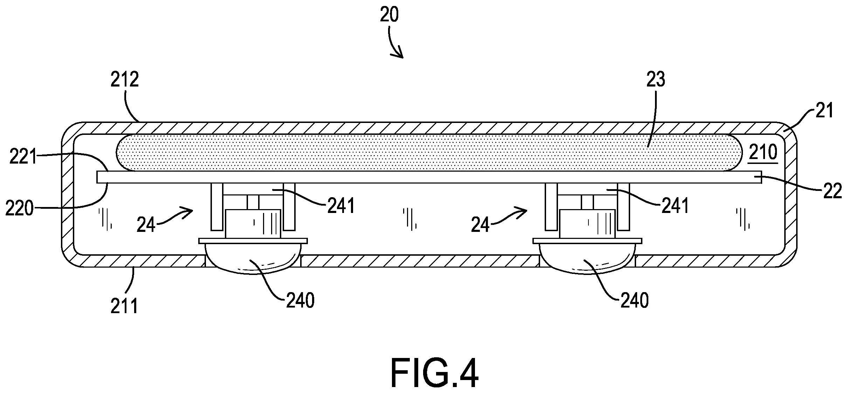

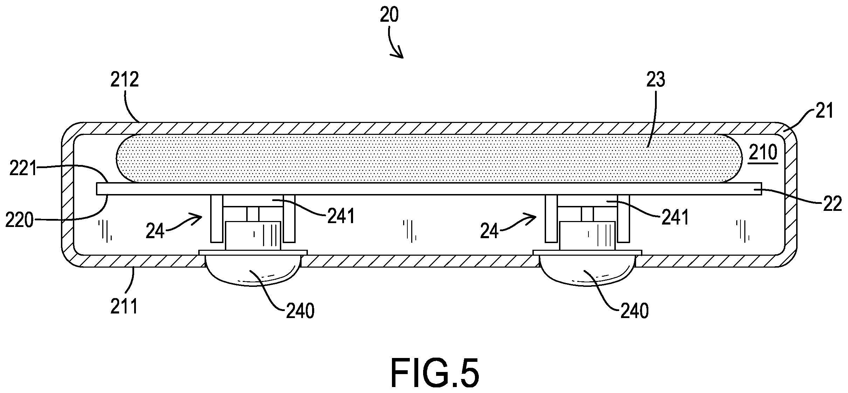

The second body 20 comprises a second shell 21, a control circuit board 22, an airbag 23 and multiple pressure sensing groups. The second shell 21 is connected to the second side of the first shell, and preferably, is pivotally connected to the first shell 11. Referring to Figs. 4 and 5, the second shell 21 contains a second accommodating space 210 inside, and the control circuit board 22 is positioned in the second accommodating space 210. The control circuit board 22 has a first surface 220 and a second surface 221 opposite to the first surface 220, with the airbag 23 positioned in the second accommodating space 210. The airbag 23 is located between the second surface 221 of the control circuit board 22 and the second shell 21 and connected to the air pump 21, with a volume of the airbag 23 adjustable according to an on/off state of the air pump 21.

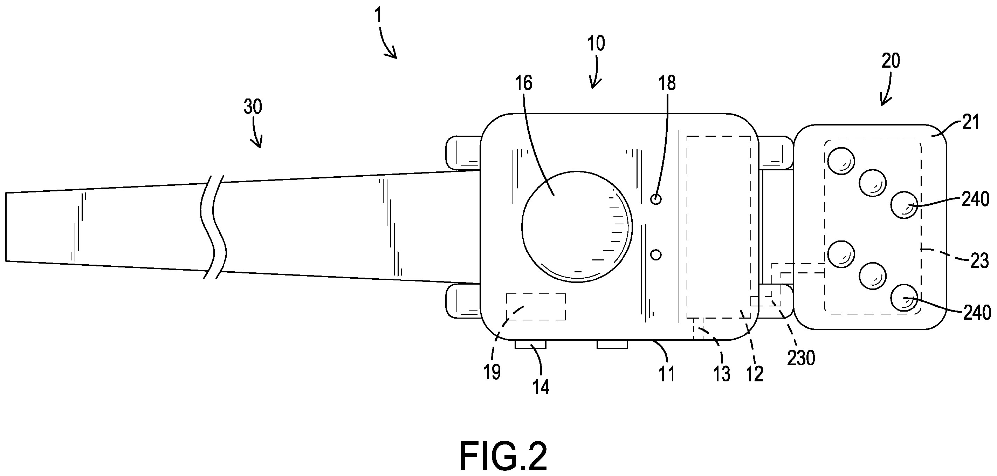

For example, referring to Figs. 1 and 2, the airbag 23 is connected to the air pump 12 by a pipe 230. When the air pump 12 is turned on, the air extracted into the air pump 12 from the channel 13 will be filled into the airbag 23 through the pipe 230, thereby inflating the airbag 23. When the air pump 12 has been turned off for a period of time, the air in the airbag will leak from the air pump 12 or the channel 13 through the pipe 230, thereby restoring the airbag to an uninflated state.

Each pressure sensing group comprises multiple pressure sensors 24 positioned on the first surface 220 of the control circuit board 22. Specifically, referring to Figs. 3 to 5, outer surfaces of the second shell 21 include an inside surface 211 and an outside surface 212, and each pressure sensor 24 comprises a contact portion 240 and a sensor body 241, with each contact portion 240 exposed from the inside surface 211 and connected with each sensor body 241. The outside surface 212 is equipped with a bracelet fixing portion 25 detachably connected with the bracelet band 30. For example, the bracelet fixing portion 25 is a bracelet buckle, and the bracelet band 30 can pass through and be buckled in the bracelet buckle to connect to the second body 20.

When the pressure-sensitive smart electronic bracelet 1 is worn on the wrist W of the user, each contact portion 240 contacts the wrist W, and a contact area between each contact portion 240 and the user (the wrist W) can be increased or decreased with the changing volume of the airbag 23. When the airbag 23 is inflated, it pushes the control circuit board 22 to move, and the contact portion 240 will therefore be further pushed out of the second accommodating space 210 (as shown in Fig. 5). The area of the contact portion 240 exposed from the inside surface 211 will increase, and the contact area between each contact portion 240 and the user will increase as well. Furthermore, pushed by the airbag 23, each contact portion 240 can more closely attach to the wrist W of the user.

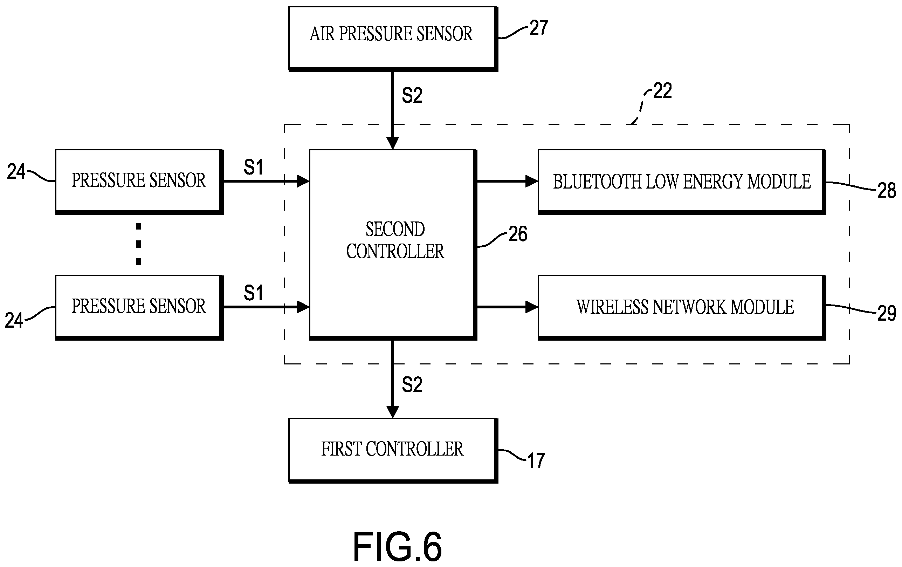

Referring to Fig. 6, each pressure sensor 24 is electrically connected to the control circuit board 22 and is adapted to sense the user's pulse beats information (known as heartbeats) to generate a pressure sensing signal S1. In particular, the control circuit board 22 is equipped with a second controller 26, which can also be a microcontroller. The second controller 26 is electrically connected to each pressure sensor 24; preferably, the second controller 24 is electrically connected to the sensor body 241 of each pressure sensor 24. The user's pulse beats information can be transmitted to each sensor body 241 through each contact portion 240, and each sensor body 241 will transmit an analog signal to the second controller 24 respectively, the analog signal being the pressure sensing signal S1. Please note that when each contact portion 240 is pushed by the airbag 23 and more closely attached to the wrist W, signal strength of the pressure sensing signal S1 generated by each pressure sensor will therefore be enhanced (compared with the uninflated state of the airbag 23).

In an embodiment of the present invention, the multiple pressure sensors 24 include multiple first pressure sensors and multiple second pressure sensors. Each first pressure sensor respectively transmits a first pressure sensing signal to the control circuit board 22, and each second pressure sensor respectively transmits a second pressure sensing signal to the control circuit board 22. That is, each pressure sensing group respectively transmits the first pressure sensing signal and the second pressure sensing signal to the control circuit board 22 (the second controller 26), and the control circuit board (the second controller 26) computes difference between each first pressure sensing signal and each second pressure sensing signal to generate multiple pressure processed signals.

In particular, each pressure sensing group includes a first pressure sensor and a second pressure sensor, and the contact portion 240 of each first pressure sensor and the contact portion 240 of each second pressure sensor are respectively located on two opposite sides of the inside surface 211 of the second shell 21. When the user wears the pressure sensitive smart electronic bracelet 1, at least one of the multiple sensing groups can correspond to a position of the user's arteries, and the contact portions 240 of the first pressure sensor of the at least one sensing group and the contact portions 240 of the second pressure sensor of the at least one sensing group can also correspond to the position of the user's arteries.

The second controller 26 is configured to compare the waveform amplitudes of the first and second pressure sensing signals of the pressure sensing group, and to designate the signal with the lesser amplitude as a reference signal.

The second controller 26 generates a pressure-processed signal by subtracting the reference signal from the other signal-typically the one with the larger amplitude-among the first and second pressure sensing signals. For instance, if the first pressure sensing signal of a given pressure sensing group has a smaller waveform amplitude, the second controller 26 designates it as the reference signal. It then subtracts the first pressure sensing signal from the second to produce the corresponding pressure-processed signal.

The second controller 26 applies the same subtraction process to other pressure sensing groups that are not aligned with the user's arteries. In these cases, however, the resulting pressure-processed signal has a value of zero. In contrast, for the pressure sensing group corresponding to the user's arteries, the pressure-processed signal yields a non-zero value, indicating the presence of arterial pressure variation.

In an embodiment of the present invention, referring to Fig. 6, the second controller 26 is connected with the first controller 17 and the air pressure sensor 27. The air pressure sensor 27 is positioned in the second accommodating space 210 and adapted to sense an air pressure inside the airbag 23 to generate an air pressure measuring signal S2 for the second controller 26. The second controller 26 transmits the air pressure measuring signal S2 to the first controller 17, so that the first controller 17 controls the air pump 12 to be turned off according to the air pressure measuring signal S2. For example, the first controller 17 presets an air pressure threshold value and determines whether an air pressure value in the airbag 23 is greater than the air pressure threshold value according to the air pressure measuring signal S2. When greater, that means the airbag 23 is filled with air, and the first controller 17 controls the air pump 12 to be turned off to stop inflating the airbag 23.

In the present embodiment, the control circuit board 22 is further equipped with at least one of a Bluetooth Low Energy module 28 (BLE) and a wireless network module 29 (such as a Wi-Fi module). The Bluetooth Low Energy module 28 and the wireless network module 29 are electrically connected to the second controller 26 respectively. The second controller 26 outputs the received pressure sensing signal S1 and the air pressure measuring signal S2 through at least one of the Bluetooth Low Energy module 28 and the wireless network module 29. For example, the second controller 26 can communicate with an AI (Artificial Intelligence) server through the Bluetooth Low Energy module 28 or the wireless network module 29 to transmit the pressure sensing signal S1 and the air pressure measuring signal S2 to the AI server.

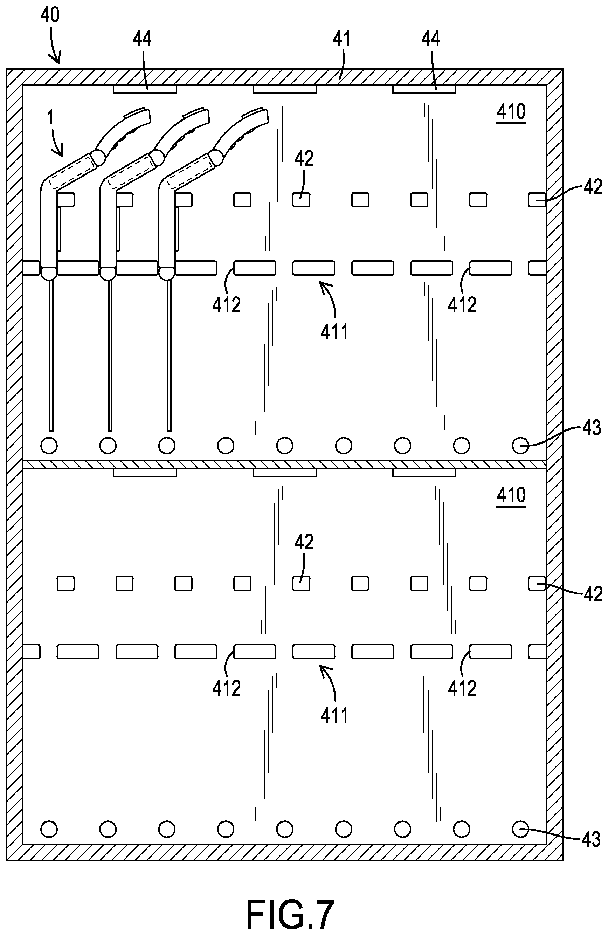

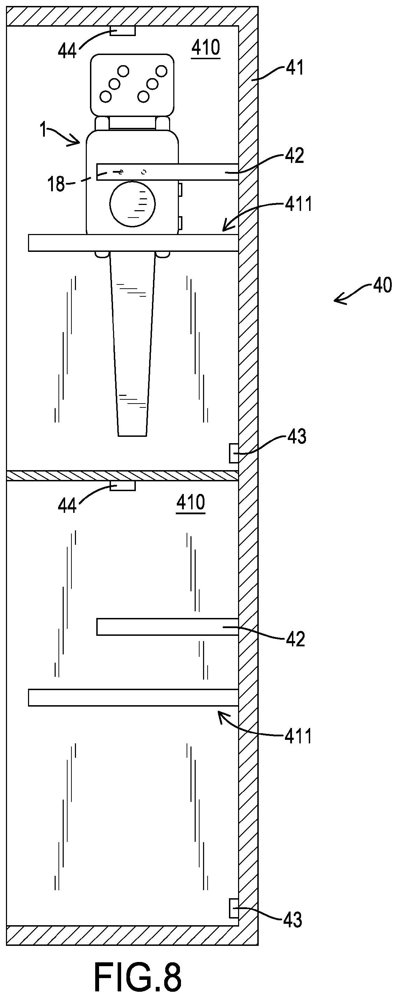

In an embodiment of present invention, the first body 10 includes at least one charging port 18 as shown in Fig. 2, exposed from the outer surface of the first shell 11. In the present embodiment, referring to Figs. 7 and 8, the pressure-sensitive smart electronic bracelet 1 can cooperate with an electronic bracelet charging cabinet 40, which comprises a cabinet housing 41, multiple charging columns 42, multiple indicating lights 43, and multiple ultraviolet (UV) lights 44. The cabinet housing 41 contains at least one bracelet accommodating space 410 inside, each bracelet accommodating space 410 equipped with a frame 411, multiple charging columns 42, multiple indicating lights 43, and multiple ultraviolet lights. The frame 411 comprises multiple slots 412, each slot 412 adapted for inserting and setting a pressure-sensitive smart electronic bracelet 1. The position of each slot 412 corresponds to the position of a charging column 42 and an indicating light 43. Therefore, when the pressure-sensitive smart electronic bracelet 1 is positioned in the electronic bracelet charging cabinet 40, the at least one charging port 18 can be electrically connected to a charging column 42 for charging. For example, the at least one charging port 18 is magnetically attracted to each charging column 42, and the indicating light 43 is adapted for reminding the user of a charging state of the pressure-sensitive smart electronic bracelet 1. The multiple ultraviolet lights are arranged to emit ultraviolet light toward the pressure-sensitive smart electronic bracelet 1 disposed within the bracelet accommodating space 410, thereby performing disinfection.

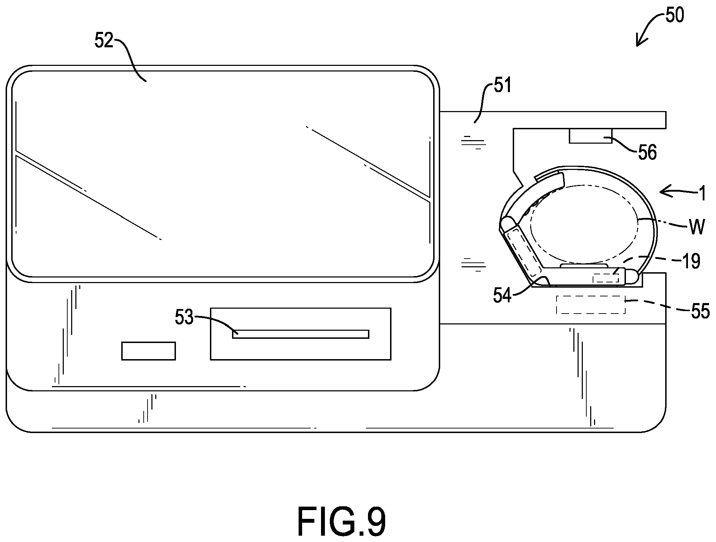

In another embodiment of the present invention, the first body 10 includes a Near-field communication Tag 19 (NFC Tag) as shown in Fig. 2. The Near-field communication Tag 19 is positioned in the first accommodating space, with an identification information stored in the Near-field communication Tag 19, wherein the identification information can be, for example, a unique identifier (UID). In the present invention, referring to Fig. 9, the pressure-sensitive smart electronic bracelet 1 can cooperate with an identity pairing device 50 comprising a shell 51, a touchscreen 52, a card slot 53 and a bracelet aligning recess 54. The touchscreen 52, the card slot 53 and the bracelet aligning recess 54 are positioned on the shell 51. The touchscreen 52 is adapted for the user to touch and operate and the card slot 53 for the user to insert a personal identity document (such as health insurance card). The pressure-sensitive smart electronic bracelet 1 is accommodated in the bracelet aligning recess 54 and then worn at a specific position on the user's wrist according to the user' operation. Furthermore, the bottom and top of the bracelet aligning recess 54 are respectively equipped with a Near-field communication Reader 55 (NFC Reader) and a lens 56. The Near-field communication Reader 55 is adapted to read the Near-field communication Tag 19 in the pressure-sensitive smart electronic bracelet 1 with the lens 56 adapted to photograph an internal space of the bracelet aligning recess 54.

A cooperative application method of the pressure-sensitive smart electronic bracelet 1 and the identity pairing device 50 is described as follows. In short, first the user inserts the personal identity document into the card slot 53 to verify identity and then positions the pressure-sensitive smart electronic bracelet 1 in the bracelet aligning recess 54. The Near-field communication Reader 55 reads the identification information in the Near-field communication Tag 19, and the identity pairing device 50 verifies the identification information and matches it with the user's identity. Then, the identity pairing device 50 activates the lens 56 and displays operation prompt information on the touch screen to prompt the user to put his/her hand into the bracelet aligning recess 54. The user can adjust his/her hand through the touch screen 52 to match the pressure-sensitive smart electronic bracelet 1 to complete aligning. Preferably, the pressure-sensitive smart electronic bracelet 1 can be connected to the personal pairing device 50 through the aforementioned Bluetooth Low Energy module 28 or the wireless network module 29. The user will be able to operate the functions of the pressure-sensitive smart electronic bracelet 1 on the identity pairing device 50, such as turning on the air pump 12 to measure the user's physiological data.

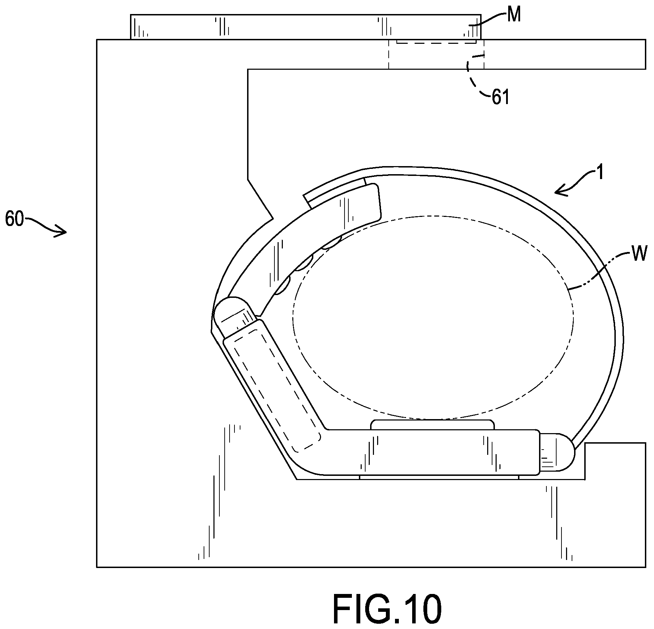

In another embodiment of the present invention, the air pump 12 is connected to the control circuit board 22. The control circuit board 22 is connected to a mobile device M with a photographing function through the Bluetooth Low Energy module 28 or the wireless network module 29 (as shown in Fig. 10), wherein the mobile device M can be a smart phone. The mobile device M is adapted to photograph the internal space of an auxiliary wearing frame 60. In particular, a first opening 61 is located on the top of the auxiliary wearing frame 60, on which the user can place the mobile device M to photograph the internal space of the auxiliary wearing frame 60 through the first opening 61.

The cooperative application method of the mobile device M, the pressure-sensitive smart electronic bracelet 1 and the auxiliary wearing frame 60 is described as follows. In short, first the user can execute an application (APP) on the mobile device M and log in to the application to verify identity. Then, the user positions the pressure-sensitive smart electronic bracelet 1 in the auxiliary wearing frame 60 and operates the application to perform the photographing function of the mobile device M. A screen of the mobile device M displays operation prompt information to prompt the user to put his/her hand into the auxiliary wearing frame 60. The user can adjust the specific position of his/her hand through the screen of the mobile device M to correspond to the pressure-sensitive smart electronic bracelet 1. Subsequently, the user can buckle the bracelet band 30 by himself/herself to complete the wearing. Preferably, the user will be able to operate the functions of the pressure-sensitive smart electronic bracelet 1 on the mobile device M, such as turning on or off the air pump 12 through the mobile device M.

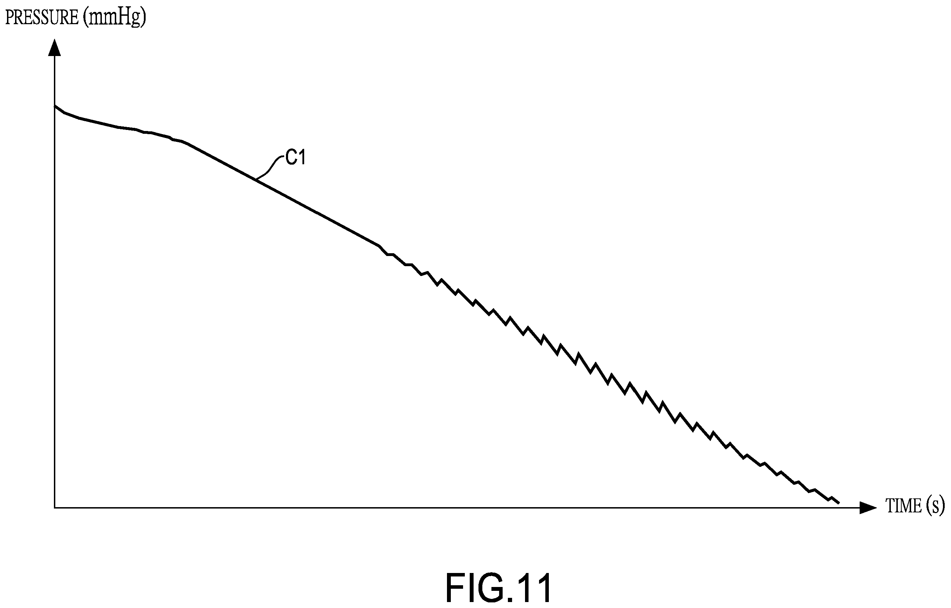

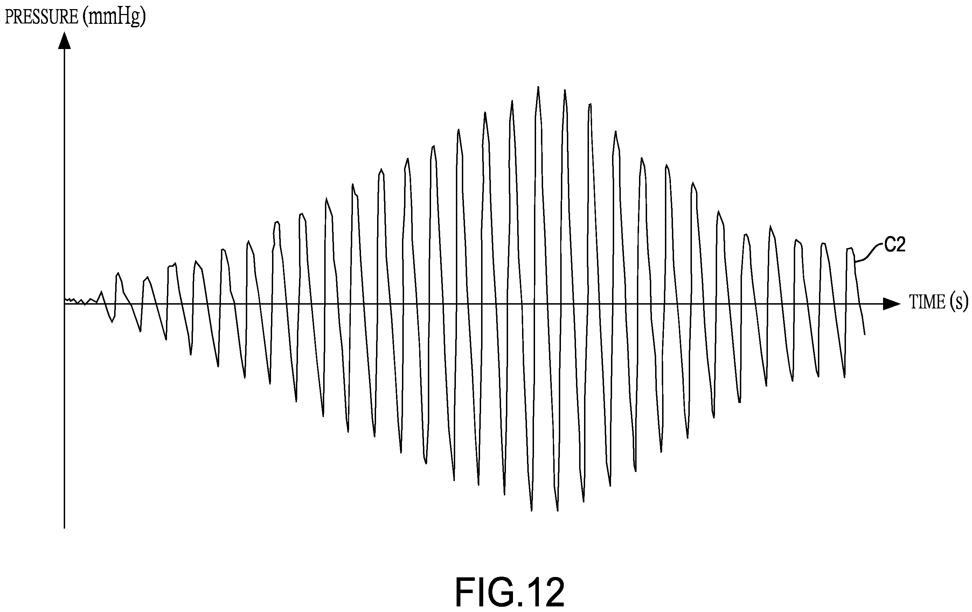

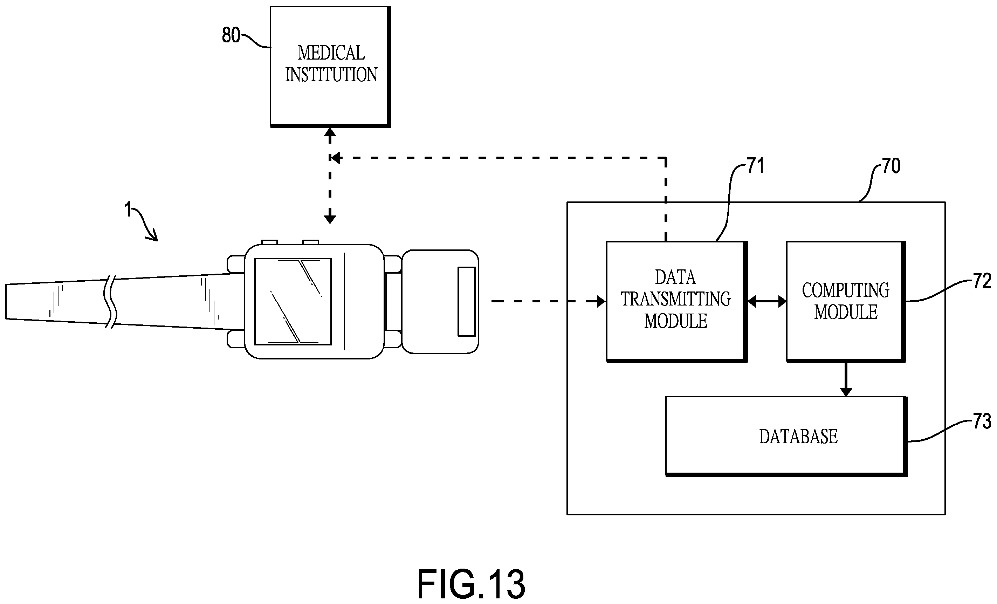

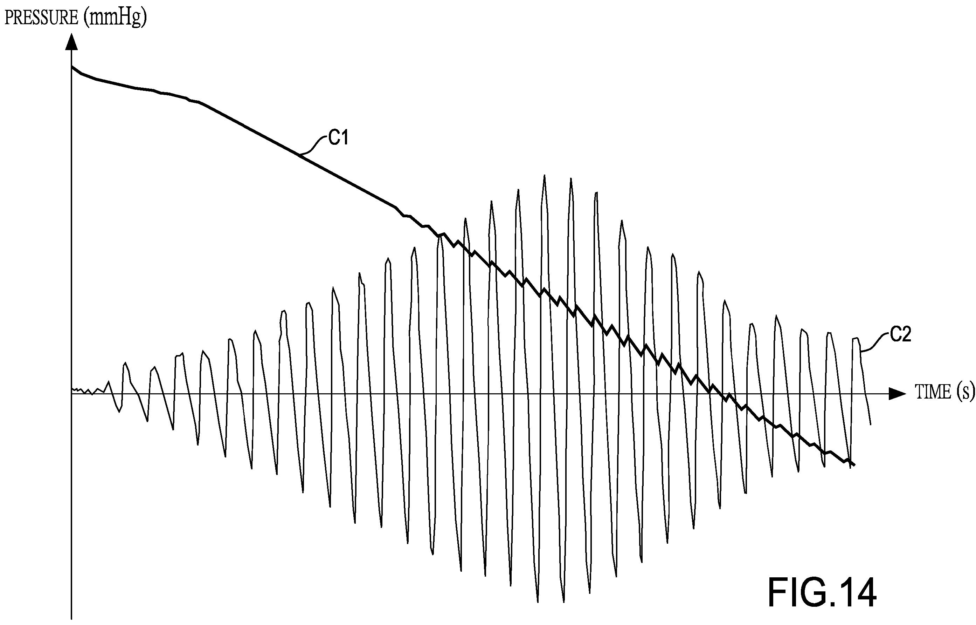

The pressure-sensitive smart electronic bracelet 1 of the present invention can perform a blood pressure measuring process and a pulse condition measuring process respectively. First, the blood pressure measuring process is described, including the following steps:
1. When the pressure-sensitive smart electronic bracelet 1 is tied to a user's wrist, the air pump 12 of the pressure-sensitive smart electronic bracelet 1 inflates the airbag 23 according to the user's operation. For example, as in the aforementioned embodiment, the user can turn on the air pump 12 through the at least one button 14 or the screen 15 to inflate the airbag, or set the air pump 12 to be automatically turned on to inflate the airbag 23 through the at least one button 14 or the screen 15.
2. When the air pump 12 stops operating and the airbag 23 deflates, the control circuit board 22 receives the pressure sensing signal S1 transmitted by each pressure sensor 24 and performs multiple signal processing processes on each pressure sensing signal S1 to obtain a pulse amplitude signal. For example, as in the aforementioned embodiment, the first controller 17 is connected to the control circuit board 22 and the air pump 12 and presets an air pressure threshold value, determining whether the air pressure value in the airbag 23 is greater than the air pressure threshold value according to the air pressure measuring signal S2. When greater, that means the airbag 23 is filled with air, and the first controller 17 controls the air pump 12 to be turned off to stop inflating the airbag 23.
   When the air pump 12 stops inflating the airbag 23, the airbag 23 deflates. During deflation of the airbag 23, the air pressure value in the air bag 23 will gradually decrease over time as shown by an air pressure change curve C1 in Fig. 11. The control circuit board 22 performs the multiple signal processing processes on each pressure sensing signal S1 to obtain a pulse amplitude signal C2 as shown in Fig. 12 during the deflation of the airbag 23, the multiple signal processing processes including at least one signal filtering process and at least one signal amplifying process.
3. Referring to Fig. 13, the control circuit board 22 (the pressure-sensitive smart electronic bracelet 1) transmits the pulse amplitude signal to an AI server 70, which extracts multiple feature point information through deep analysis and computation. The multiple feature point information include systolic blood pressure information and diastolic blood pressure information. For example, the AI server 70 comprises a data transmitting module 71, a computing module 72 and a database 73, the computing module 72 connected with the data transmitting module 71 and the database 73. The data transmitting module 71 is adapted to receive information transmitted by the pressure-sensitive smart electronic bracelet 1 or to output information. The computing module 72 is built in with multiple algorithms, analyzing and computing the information transmitted by the pressure-sensitive smart electronic bracelet 1. For example, referring to Fig. 14, the computing module 72 superimposes the air pressure change curve C1 and the pulse amplitude signal to obtain multiple intersection points, the multiple intersection points being the multiple feature point information.

Furthermore, the computing module 72 performs deep analysis and computation on the signals transmitted by the pressure-sensitive smart electronic bracelet 1 to obtain multiple analysis results. Then, the data transmitting module transmits the multiple analysis results to the pressure-sensitive smart electronic bracelet 1 or a medical institution 80, enabling the user or medical staff to promptly know the user's physiological state. The database 73 is adapted to record information transmitted by the pressure-sensitive smart electronic bracelet 1 or computed by the computing module 72.

Steps of the pulse condition measuring process are partially same as the steps of the blood pressure measuring process with the difference described as follows. When the air pump 12 stops operating and the airbag 23 is deflating, the control circuit board 22 receives the pressure sensing signal S1 transmitted by each pressure sensor 24 and performs multiple signal processing processes on each pressure sensing signal S1 to obtain a pulse wave signal. The control circuit board performs Fourier transform on the pulse wave signal to obtain a high-frequency signal and a low-frequency signal and transmits the pulse wave signal, the high-frequency signal and the lower-frequency signal to the AI server 70, which extracts multiple feature wave information through deep analysis and computation.

The pressure-sensitive smart electronic bracelet 1 of the present invention comprises a first body 10 and a second body 20. Contrary to the prior art, the present invention increases the accommodating space of physiological data sensors, eliminating limits on the types of physiological data sensors. The second body 20 is equipped with multiple pressure sensor 24 with each pressure sensor 24 comprising a contact portion 240. When the user wears the pressure-sensitive smart electronic bracelet 1, each pressure sensor 24 receives the user's pulse beats information through the contact portion 240 to accordingly generate a pressure sensing signal. In addition, an airbag 23 is positioned in the second body 20, and with the airbag inflated, each contact portion 240 can further protrude from the outer surface of the second body 20 to increase the contact area with the user, pushing each contact portion 240 by the airbag 23 to be more closely attached to the user. The conventional electronic bracelet only senses pulse data through the optical sensor with its transmitting terminal (or a receiving terminal) prone to blockage by objects, such as dust, moisture, etc., resulting in inaccuracy of pulse data sensed by the optical sensor. Contrary to the prior art, the pressure-sensitive smart electronic bracelet 1 of the present invention measures the user's pulse data through physical contacting, effectively improving the accuracy of measuring physiological data collected through physical means such as blood pressure and pulse condition.

The pressure-sensitive smart electronic bracelet 1 of the present invention features unique physical sensing technology and can instantly detect the user's vital signs, including blood pressure, pulse condition and other physiological indicators. The pressure-sensitive smart electronic bracelet 1 transmits the obtained data to the AI server 70, which performs deep analysis and computation to obtain multiple analysis results, fed back to the user and also simultaneously provided to relevant medical institutions. Therefore, the medical staff can promptly know the user's physiological state, facilitating effective medical treatment and advice.

While the present invention has been described above in a preferred embodiment, it is not intended to limit the invention. Those skilled in the art may make changes and modifications without departing from the spirit and scope of the invention. Therefore, the scope of protection of the invention shall be determined by the appended patent claims.

## Claims

1. A pressure-sensitive smart electronic bracelet (1), **characterized in that** the pressure-sensitive smart electronic bracelet (1) comprises:
a first body (10), comprising:
a first shell (11) with a first side and a second side opposite to the first side, the first side connected with a bracelet band (30) and the first shell (11) containing a first accommodating space; and
an air pump (12) positioned in the first accommodating space; and
a second body (20), comprising:
a second shell (21) connected with the second side of the first shell (11) with a second accommodating space (210) inside the second shell (21);
a control circuit board (22) positioned in the second accommodating space (210) and comprising a first surface (220) and a second surface (221) opposite to the first surface (220);
multiple pressure sensing groups, each pressure sensing group comprising multiple pressure sensors (24) positioned on the first surface (220) of the control circuit board (22), each pressure sensor (24) electrically connected to the control circuit board (22) with a contact portion (240) thereof exposed from an outer surface of the second shell (21), and each pressure sensor (24) detects a user's pulse beats information to accordingly generate a pressure sensing signal (S1); and
an airbag (23) positioned in the second accommodating space (210), located between the second surface (221) of the control circuit board (22) and the second shell (21), and connected to the air pump (12).

2. The bracelet as claimed in claim 1, wherein the first body (10) comprises:
at least one button (14) positioned on an outer surface of the first shell (11);
a screen (15) positioned in the first accommodating space with a display surface of the screen (15) exposed from the outer surface of the first shell (11);
an optical sensor (16) positioned in the first accommodating space with a detecting surface of the optical sensor (16) exposed from the outer surface of the first shell (11); and
a first controller (17) positioned in the first accommodating space and electrically connected with the at least one button (14), the screen (15), the optical sensor (16), and the air pump (12).

3. The bracelet as claimed in claim 2, wherein
the control circuit board (22) comprises a second controller (26) connected with the first controller (17); and
an air pressure sensor (27) is positioned in the second accommodating space (210) and electrically connected to the second controller (26);
the air pressure sensor (27) detects an air pressure inside the airbag (23) to generate an air pressure measuring signal (S2) for the second controller (26).

4. The bracelet as claimed in claim 3, wherein
the control circuit board (22) comprises at least one of a Bluetooth Low Energy module (BLE) (28) and a wireless network module (29) electrically connected to the second controller (26) respectively;
the second controller (26) receives and outputs each pressure sensing signal (S1) and the air pressure measuring signal (S2) through the at least one of the Bluetooth Low Energy module (28) and the wireless network module (29).

5. The bracelet as claimed in claim 3, wherein the second controller (26) transmits the air pressure measuring signal (S2) to the first controller (17), and the first controller (17) controls the air pump (12) to be turned off according to the air pressure measuring signal (S2).

6. The bracelet as claimed in claim 1, wherein
the second shell (21) comprises an outside surface (212) and an inside surface (211);
a bracelet fixing portion (25) is positioned on the outside surface (212) of the second shell (21) for detachable connection to the bracelet band (30);
the contact portion (240) of each pressure sensor (24) is exposed from the inside surface (211) of the second shell (21);
each pressure sensing group comprises a first pressure sensor and a second pressure sensor, the contact portion (240) of each first pressure sensor and the contact portion (240) of each second pressure sensor are respectively located on two opposite sides of the inside surface (211) of the second shell (21).

7. The bracelet as claimed in claim 6, wherein each pressure sensing group respectively transmits a first pressure sensing signal and a second pressure sensing signal to the control circuit board (22), and the control circuit board (22) computes difference between each first pressure sensing signal and each second pressure sensing signal to generate multiple pressure processed signals.

8. The bracelet as claimed in claim 1, wherein the first body (10) comprises at least one charging port (18) exposed from the outer surface of the first shell (11) to be electrically connected with a charging column (42) of an electronic bracelet charging cabinet (40).

9. The bracelet as claimed in claim 1, wherein a Near-field communication Tag (19) is positioned in the first accommodating space of the first body (10) and readable by a Near-field communication Reader (55) of an identity pairing device (50).

10. The bracelet as claimed in claim 1, wherein
the air pump (12) is connected to the control circuit board (22), and the control circuit board (22) is connected with a mobile device (M) with a photographing function;
the mobile device (M) photographs an internal space of an auxiliary wearing frame (60) and controls the air pump (12) to be turned on or off.

11. An application method of the pressure-sensitive smart electronic bracelet (1) as claimed in any one of claims 1 to 10, **characterized in that** the application method comprises:
when the bracelet is tied to a user's wrist, the air pump (12) of the bracelet inflates the airbag (23) according to the user's operation;
when the air pump (12) stops operating and the airbag (23) is deflating, the control circuit board (22) receives the pressure sensing signal (S1) transmitted by each pressure sensor (24) and performs multiple signal processing processes on each pressure sensing signal (S1) to obtain a pulse amplitude signal, and the control circuit board (22) transmits the pulse amplitude signal to an AI (Artificial Intelligence) server (70), which extracts multiple feature point information through deep analysis and computation;
the multiple feature point information includes systolic blood pressure information and diastolic blood pressure information.

12. The application method as claimed in claim 11, wherein the multiple signal processing processes include at least one signal filtering process and at least one signal amplifying process.

13. An application method of a pressure-sensitive smart electronic bracelet (1) as claimed in any one of claims 1 to 10, **characterized in that** the application method comprises:
when the bracelet is tied to a user's wrist, the air pump (12) of the bracelet inflates the airbag (23) according to the user's operation;
when the air pump (12) stops operating and the airbag (23) is deflating, the control circuit board (22) receives the pressure sensing signal (S 1) transmitted by each pressure sensor (24), performs multiple signal processing processes on each pressure sensing signal (S1) to obtain a pulse wave signal, performs Fourier transform on the pulse wave signal to obtain a high-frequency signal and a low-frequency signal, and transmits the pulse wave signal, the high-frequency signal, and the lower-frequency signal to an AI server (70), which extracts multiple feature wave information through deep analysis and computation.
